Europäisches Patentamt

European Patent Office

Office européen des brevets

(19)

(11) Publication number: **0 429 866 B1**

(12) **EUROPEAN PATENT SPECIFICATION**

(45) Date of publication of patent specification: **25.05.94**

(51) Int. Cl.5: **A61M 5/142**, G01L 9/00, G01L 19/12

(21) Application number: **90120710.0**

(22) Date of filing: **29.10.90**

(54) **Apparatus and method for measuring pressure in a fluid line for detecting an occlusion in said fluid line.**

(30) Priority: **02.11.89 US 430809**

(43) Date of publication of application:
**05.06.91 Bulletin 91/23**

(45) Publication of the grant of the patent:
**25.05.94 Bulletin 94/21**

(84) Designated Contracting States:
**DE FR GB**

(56) References cited:
**EP-A- 0 040 592**
**EP-A- 0 283 614**
**US-A- 4 373 525**
**US-A- 4 690 673**

(73) Proprietor: **IVAC CORPORATION**
**10300 Campus Pt. Drive**
**San Diego, CA 92121(US)**

(72) Inventor: **Daoud, Adib G.**
**4223 Middlesex Drive**
**San Diego, California 92116(US)**
Inventor: **Horres, Russell C.**
**13071 Via Grimaldi**
**Del Mar, California 92014(US)**
Inventor: **Everhart, Howard R.**
**3808 Mt. Ainsworth Avenue**
**San Diego, California 92111(US)**

(74) Representative: **Baillie, Iain Cameron et al**
**c/o Ladas & Parry**
**Altheimer Eck 2**
**D-80331 München (DE)**

## Description

Field of the Invention:

This invention relates generally to a device for detecting occlusion in a fluid line, and more particularly relates to detection of occlusion in a fluid line upstream or downstream of a peristaltic type pump used for infusion of intravenous solutions to a patient.

Description of Related Art:

Various devices have been used for infusing intravenous fluids to a patient. In order to gain more control over the rate of fluid flow than is generally available with gravity feed intravenous (IV) administration sets, various types of pumps and controllers have been utilized. A peristaltic type of pump has been found to be advantageous in allowing the pumping of intravenous fluid through conventional, flexible fluid lines without fluid contact. It has been found that the operation of peristaltic pumps also allow for a wide variety of control and sensing of patency and fluid flow conditions. Important data can be gathered by monitoring fluid pressure changes within the intravenous fluid line.

The incorporation of a pressure sensing strain gauge assembly in a peristaltic pump in order to monitor dimensional changes in the outer diameter of an intravenous tube as an indication of fluid pressure changes in the tube is known from U. S. Patent No. 4,690,673. In the construction of such a pressure sensing assembly, the strain gauge is attached to a strain beam which is part of an assembly mounted on a fixed mounting block to press with a generally constant displacement of the fluid line.

It would be desirable to provide an apparatus for monitoring occlusion in a fluid line upstream or downstream of a peristaltic pump mechanism, or otherwise detecting fluid pressure changes in the fluid line for detecting changes in the rate of flow due to partial closure of the line, leaks in the line, or other such problems with a pressure sensor which presses against the line with a certain degree of displacement of the fluid line only during a limited portion of the duty cycle of the pump. Such a moving pressure sensor can avoid interfering with the fluid flow during the portion of the duty cycle when the pressure sensor is not pressing against the fluid line, and would allow for periodic calibration of the pressure sensor as well. Coordination of the movement of the pressure sensor with cam follower fingers of a peristaltic pump would be desirable in order to avoid interfering with the operation of the pump, while enhancing the accuracy of pressure measurements obtained. The present invention addresses these needs.

## SUMMARY OF THE INVENTION

The present invention provides an improved system for detecting pressure and occlusion in a fluid line being operated upon by a positive displacement, peristaltic pump having several cam follower fingers pressing against the fluid sequentially to force fluid through the line peristaltically. A sensor follower finger is mounted to the cam shaft of the pump among the other peristaltic fingers in order to cyclicly press against the fluid line with a certain degree of force and displacement as the cam shaft rotates. The sensor follower finger includes a strain gauge mounted on the sensor finger to generate a signal indicating the degree of force being applied by the sensor finger against the tubing, and a signal processor unit which receives this force signal determines the pressure within the fluid line based upon the signal. The signal processor compares the measured pressure with an upstream reference value during an upstream pressure measurement mode, and compares the measured pressure with a downstream reference value during a downstream pressure measurement mode. An alarm may also be provided for indicating if there is an occlusion condition either upstream or downstream when the measured pressure varies significantly from these reference values. The force sensor is preferably a strain gauge mounted on a portion of the sensor finger which is subject to strain forces as the sensor finger presses against the tubing.

Briefly, and in general terms, an apparatus for measuring pressure within a flexible tubing being operated upon by a peristaltic pump having a plurality of cam follower fingers includes a sensor finger periodically pressing against the tubing wall with a predetermined displacement of the tubing wall, preferably without occluding the fluid line, a sensor for measuring the force exerted by the sensor finger on the tubing wall and generating a force signal indicative of the force; and a signal processing unit for determining the measured pressure based upon the force signal, and for comparing the measured pressure with upstream and downstream pressure reference values during upstream and downstream modes, respectively.

In a preferred embodiment, the pressure measurement apparatus includes an alarm for indicating an occlusion condition, based upon the comparison of the measured fluid line pressure with either the upstream or downstream reference pressure values. The force sensor also preferably comprises a strain gauge mounted on a portion of the sensor finger for measuring the force exerted by the sensor finger on the tubing wall, and the strain gauge is most preferably placed within an aperture in the portion of the sensor finger which engages the tubing wall.

Other aspects and advantages of the invention will become apparent from the following detailed description and the accompanying drawings illustrating by way of example the features of the invention.

## BRIEF DESCRIPTION OF THE DRAWINGS

FIGURE 1 is a simplified sectional view of the peristaltic finger mechanism operating upon a fluid line, and showing the placement of the sensor of the invention;

FIG. 2 is a side elevational view of a sensor follower according to the invention journalled to a cam shaft drive;

FIG. 3 is a view similar to Fig. 1 showing the sensor follower in position during an upstream mode of the peristaltic pump;

FIG. 4 is a schematic diagram of the relationship of the sensor follower to flexible tubing within the peristaltic pump; and

FIG. 5 is a flow chart illustrating the operation of the signal processing unit.

## DETAILED DESCRIPTION OF THE INVENTION

As is shown in the drawings for purposes of illustration, the invention is embodied in an apparatus and method for measuring pressure in a fluid line received in a peristaltic pump, for determining whether the fluid line is occluded upstream or downstream of the pump mechanism. The apparatus comprises a sensor follower finger journalled to a rotating cam shaft and centrally located among a plurality of cam follower fingers journalled to the cam shaft. The sensor follower finger is adapted to press against the flexible tubing wall at a generally central portion of the segment of fluid line received in the peristaltic pump, intermediate the upstream end and downstream end cam followers. The sensor follower is adapted to cyclically press against the segment of tubing for a portion of the duty cycle pump with predetermined displacement of the tubing wall without occluding the fluid line. A sensor associated with the sensor follower finger measures the force exerted by the sensor follower finger on the tubing segment, and signal processing unit compares the upstream and downstream pressures with reference values to determine whether occlusion has occurred upstream or downstream from the pump.

In accordance with the invention, there is therefore provided an apparatus for measuring pressure within a compressible pumping segment of a fluid line having a flexible tubing wall, including a peristaltic pump mechanism for receiving the pumping segment, the peristaltic pump mechanism having a rotating camshaft and a plurality of cam follower fingers journalled thereto and including an upstream end cam follower finger and a downstream end cam follower finger adapted to sequentially press against the pumping segment producing cyclically recurring upstream and downstream pressure communication modes of a duty cycle of the pump in which a central portion of the pumping segment is alternately in communication with only an upstream portion of the fluid line and only a downstream portion of the fluid line, respectively, the apparatus comprising a sensor follower finger journalled to the camshaft adjacent the central portion of the pumping segment and intermediate the upstream end cam follower and the downstream end cam follower, and adapted to cyclically press against the pumping segment for a portion of the duty cycle with a predetermined displacement of the tubing wall without occluding the fluid line; sensor means associated with the sensor follower finger for generating a force signal indicative of the force exerted by the sensor follower finger in pressing against the pumping segment; and signal processing means responsive to the force signal for determining a measured pressure value based upon the force signal, for comparing the measured pressure with an upstream pressure threshold value in the upstream pressure communication mode, and adapted to generate an upstream occlusion error signal when the measured pressure ie less than the upstream pressure threshold value, and for comparing the measured pressure with a downstream pressure threshold value in the downstream pressure communication mode and adapted to generate a downstream occlusion error signal when the measured preSSure is greater than the downstream pressure threshold value.

The invention also provides a method for measuring pressure within a pumping segment of a fluid line having a flexible tubing wall, comprising the steps of periodically compressing the pumping segment with at least one finger member with a predetermined amount of displacement of the flexible tubing wall without occluding the fluid line; measuring a force parameter exerted by the finger member on the flexible tubing wall; generating a force signal indicating the measured force parameter; and determining fluid pressure within the tubing responsive to the force signal.

As is shown in the drawings, a peristaltic pump mechanism 10 is adapted to receive a flexible fluid tubing 12, to force fluid through the tubing in the direction of the arrow 14. The fluid tubing includes a compressible pumping segment 16 from approximately the location A to the location B, between the inlet end 18 of the tubing and the outlet end 20. Although the tubing is typically continuous, and flexible throughout its length, it is also possible that an inflexible tubing could be used, with an intermediate compressible pumping segment spliced into the inflexible tubing at the inlet and outlet ends of the compressible pumping segment, to allow a peristaltic pump mechanism to act upon the pumping segment. The peristaltic pump mechanism also includes a portion of the pump housing 22 adjacent to one side of the flexible tubing, and typically several peristaltic cam follower fingers 23 journalled to a camshaft 24, which drives the peristaltic cam follower fingers to sequentially press against the flexible tubing wall of the pumping segment to force the fluid in the tubing to flow, by peristaltic movement. As the cam rotates about its off center axis of rotation 26, the cam surface 28 precesses around the axis, causing the cam follower fingers to sequentially press against and move away from the tubing wall.

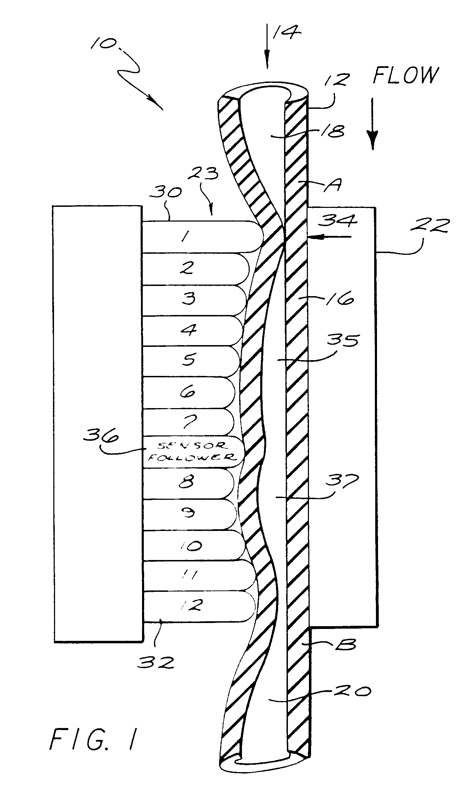

The farthest upstream cam follower finger 30 identified in Figure 1 as finger No. 1 out of a sequence of 12 cam follower fingers, and the farthest downstream cam follower finger 32, identified as finger No. 12, form the outside end fingers of the peristaltic pump mechanism, and are designed to sequentially press against and occlude the fluid line, to force fluid through the tubing. In Figure 1, the uppermost finger 30 is shown as occluding fluid tubing at the location 34, and finger No. 2, finger No. 3, finger No. 4, and so on sequentially press against the tubing and occlude the fluid flow at the area of contact, forcing fluid along the tubing. As the point of occlusion moves sequentially downstream past the central area of the cam follower fingers, the downstream portion of the fluid tubing will be sealed off from communication with the central portion of the fluid tubing, where the non-occluding sensor follower finger 36 is approximately located. At this point, fluid pressure from the upstream portion of the tubing is in fluid communication through the fluid tubing portion 35 with the tubing area adjacent the sensor follower finger. As the point of occlusion shifts to the upstream fingers, the downstream fingers culminating in finger 32 release, opening the central area adjacent the sensor follower to fluid communication with the downstream portion of the tubing 20 through the area 37 just downstream of the sensor follower. Thus, an upstream mode of communication with the central area of the tubing can be defined with relation to the point of occlusion, so that when the peristaltic point of occlusion is downstream from the sensor follower and the fluid line is opened to communication upstream of the sensor follower, the peristaltic pump mechanism is an upstream mode of pressure communication, and when the point of occlusion is upstream of the sensor follower and the downstream portion of the tubing is open to communicate pressure to the sensor follower finger, the apparatus is in a downstream mode of pressure communication.

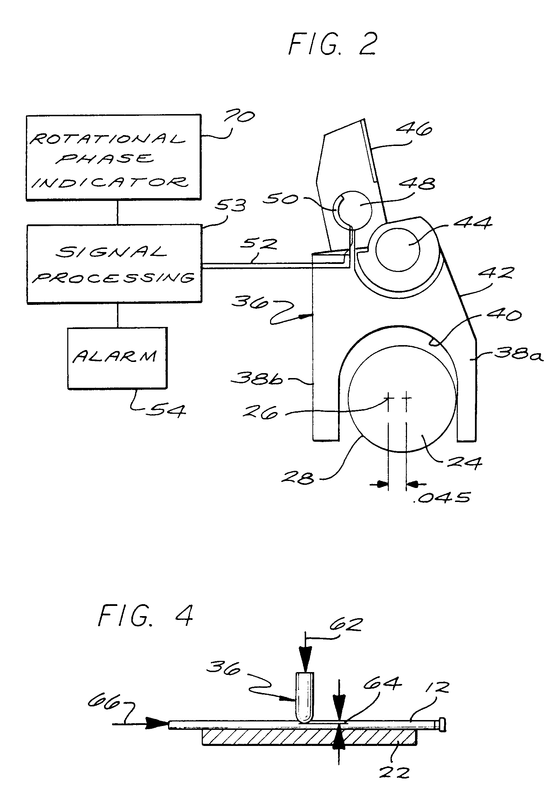

Referring to Fig. 2, the sensor follower finger 36 is generally of the same shape and construction as that of the other cam follower fingers. However, the cam follower fingerS are typically made of acetal resin or other types of engineering plastics, but the sensor follower finger is preferably made from Invar, an iron-nickel alloy having a low coefecient of thermal expansion, which is often used in precision instruments. Other metals or rigid polymers with similar characteristics may also be used. The sensor follower finger includes cam follower arms 38a, 38b adapted to cooperate within and follow the rotation of the cam drive, and having an interior U-shaped cam follower section 40, in the main body portion 42. The sensor follower finger includes a pivot aperture 44, so that the finger can be mounted to pivot in response to the cam drive, to move the tube pressing arm 46 to press against and move away from the flexible tubing. The tube pressing arm preferably includes a protective aperture 48 containing a strain gauge mounted therein to monitor the force applied by the tube pressing arm against the flexible tubing. The strain gauge is electrically connected by the wire 52 to a signal processing unit 53, typically a microprocessor based unit or computer, which receives the force signal output from the strain gauge, and which in turn generates an occlusion error signal which may drive an alarm 54, as will be explained hereinafter.

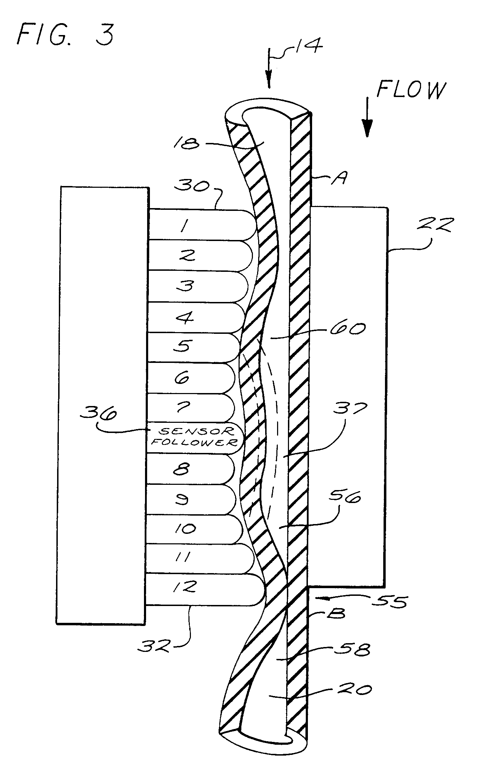

With reference Figs. 1 and 3, the sensor follower finger is driven with a cam that is different from the cam sections of the other cam follower fingers, so that the sensor follower finger will not occlude the tubing, but will contact the tubing wall in order to press against the wall with a limited displacement of the tubing wall. Furthermore, the cam for this sensor follower finger is preferably formed so that if the follower No. 1 is occluding the tubing, the sensor follower will be in contact with and pressing against the tubing, so that if the tubing is occluded downstream the sensor will reflect the occlusion in the output force signal. When the furthest downstream follower No. 12 occludes the tubing the sensor follower will again be in contact with the tubing so that if, for example, an upstream roller clamp is closed, a negative pressure will occur, which will result in a vacuum within the compressible segment of the tubing, the tubing will collapse, and the sensor follower will indicate an upstream occlusion.

With reference to Fig. 3, it can be seen that when the point of occlusion 55 is at about the location of the furthest downstream finger No. 12, the portion of the fluid line upstream 56 of the fluid line is in pressure communication with the sensor follower finger, and the downstream portion of the fluid line 58 is closed off. A vacuum 60 created by upstream occlusion, such as a roller clamp on the fluid line being closed, or some other blockage in the line would allow a vacuum to build up within the compressible pumping segment as the fluid is forced downstream by the peristaltic pump mechanism.

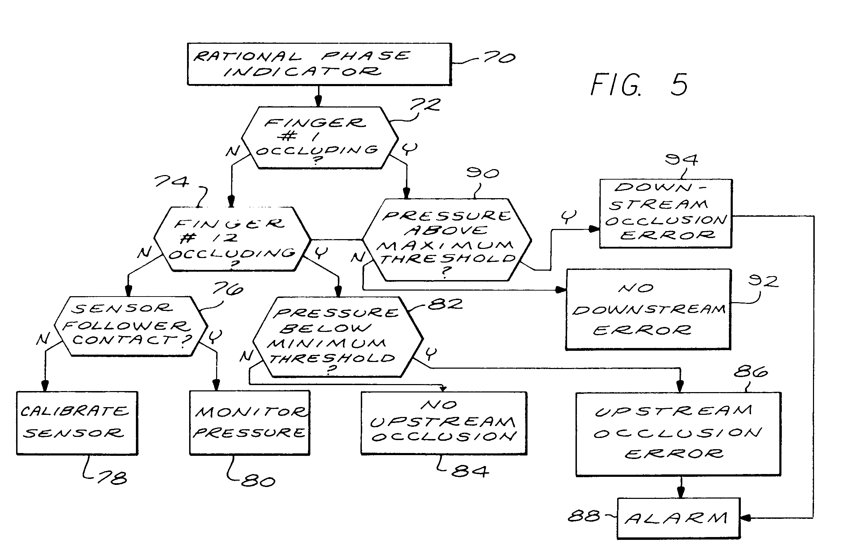

In the following discussion of determination of fluid line pressure based upon displacement of a portion of flexible tubing by a sensor follower arm and the measured force applied by the arm on the tubing, reference is made to the simplified schematic diagram shown in Fig. 4. A non-occluding sensor follower arm or blade 36 is shown as being directed against the fluid line 12 with a degree of force (in grams) and in a direction indicated by the vector 62. The blade displacement 64 of the tubing wall is in opposition to the applied pressure 66 within the fluid.

Accurate correlation between blade force against tubing (sensor response) and tubing internal pressure was found to require the use of a substantial blade displacement value, ideally in the range of from 50 to 70 mils. However, an excessive displacement should not restrict flow within the tubing. Force relaxation and load hysteresis tests further show the tube stiffness characteristics to be significantly time dependent, so that accurate correlations between sensor response and tubing internal pressure in practice should allow for execution of a frequent tubing calibration routine with actual operating flow rates and operating temperatures. Zeroing of the sensor follower finger force measurement is made possible by movement of the sensor follower finger away from the tubing wall during a portion of the duty cycle of the peristaltic pump.

Tests performed with various tubing vacuum levels simulating an upstream occlusion condition showed fairly linear results for blade force versus displacement, and internal vacuum levels in the tubing ranging from 0 to about 5 psig vacuum. At a vacuum level of about 7.5 psig, blade force levels fall off dramatically. Typically somewhere between 7.5 psig and 10 psig vacuum levels, the tube completely buckles or collapses under the action of the internal vacuum. In this state, the tubing wall becomes completely flattened and loses contact all together with the sensor follower blade.

An empirically derived equation was established for fluid pressure as a function of blade displacement, tube force and a tube bending stiffness parameter. The equation provides a reasonably close prediction of fluid pressure from tube force test data acquired for displacements ranging from 0.10 cm (40 mils) to 0.20 cm (80 mils), tube internal pressures ranging from 0 to 10 psig, tube durometers ranging from 45a to 55a, and wall thicknesses ranging from 34 to 38 mils.

Pressure determination is based upon deflection of the tubing wall, blade force, and a tube bending stiffness parameter which can be determined experimentally or from information from the supplier, according to the following equation:

$$ P = \frac{F_T/d \quad - \quad (K_1)_c}{a \quad + \quad b/(K_1)_c} $$

Where d = deflection of tube wall, in mils;
$F_T$ = blade force to deform the wall, in grams;
P = tube internal pressure in psig; and
$K_{1\,c}$ = a bending stiffness parameter.

$K_{1c}$ is proportional to $E_h^3$, where E is the modulus of elasticity in psi, and h is wall thickness in mils. The variable "a" was empirically determined to be 0.0790, and "b" was empirically determined to be 0.0478 for the test apparatus. The derived stiffness parameter $K_{1c}$ was found to typically range approximately between 1.33 and 2.42.

In the operation of the signal processing unit, generally described in the flow chart shown in Fig. 5, the signal processing unit ideally receives input from a rotational phase indicator 70, which may take the form of an optical flag assembly which interrupts a photoelectric beam, either in conjunction with the cam drive itself or a motor (not shown) which drives the rotation of the cam shaft. With a signal from the rotational phase indicator, the signal processor can identify which of the cam follower fingers is occluding the fluid line at any given moment during the duty cycle of the pump. A determination is made at 72 whether the most upstream cam follower finger is occluding the fluid line. If it is not, the same decision is made at 74 concerning the most downstream cam follower finger, finger No. 12. If finger No. 12 is not occluding the tubing, a determination is made at 72 as to whether the sensor follower is contacting the tubing, and if it is not, since the strain gauge will then be generating a signal which correlates with zero pressure, a calibration of the sensor at 78 can be made. Otherwise, if the sensor follower is contacting the tubing, the fluid pressure can be monitored at 80. When finger No. 12 is occluding the fluid line, a determination can be made at 82 whether the measured pressure determined by the signal processing unit is below a minimum threshold. A typical minimum pressure threshold would be zero, so that if the pressure level were determined to be above this level, at 84, there would not be the indicated upstream occlusion, and if a pressure less than zero were detected this would give an indication at 86 of an upstream occlusion error condition, which may also result in an alarm signal to the alarm 88. Similarly, if it is determined that finger No. 1 is occluding the fluid line, it can be determined at 90 whether the measured pressure is above a maximum threshold. If the measured pressure is less than the maximum pressure threshold, such as for example 10 psig, then the determination at 92 would be of no downstream occlusion. Otherwise, if the measured pressure were above this maximum threshold, the signal processing unit would generate a downstream occlusion error signal, and the signal processing unit may also generate an alarm signal to activate the alarm 88.

In view of the foregoing, it has been demonstrated that the system of the invention for measuring pressure within a fluid line can be used for detecting occlusion within the fluid line either upstream or downstream from a peristaltic pump mechanism operating on the fluid line. It is also significant that the sensor follower finger is adapted for movement correlated with that of the other cam follower fingers in order to avoid restriction of fluid flow, and to allow periodic calibration of the sensor.

Although specific embodiments of the invention have been described and illustrated, it is clear that the invention is susceptible to numerous modifications and adaptations within the ability of those skilled in the art and without the exercise of the inventive faculty. Thus, it should be understood that various changes in form, detail and use of the present invention may be made without departing from the invention as claimed in the claims 1 to 11.

## Claims

1. An apparatus for measuring pressure within a fluid line having a pumping segment (16) with a flexible tubing wall (12), including a peristaltic pump mechanism (10) for receiving said pumping segment, said peristaltic pump mechanism having a plurality of peristaltic fingers (23) including an upstream end finger (30) and a downstream end finger (32) adapted to sequentially press against said pumping segment producing cyclically recurring upstream and downstream pressure communication modes of a duty cycle of said pump in which a portion of said pumping segment between said end fingers is alternatively in communication with only an upstream portion of said fluid line and only a downstream portion of said fluid line respectively, and a processor (53) for receiving a force signal in said upstream pressure communication mode and for receiving said force signal in said downstream pressure communication mode and adapted to generate an occlusion signal based on the values of said force signals

characterized in that

a sensor follower finger (36) is disposed between said upstream end finger and said downstream end finger, being adapted to cyclically press against said pumping segment for a portion of said duty cycle to displace the tubing wall of said segment by a predetermined amount;

a gauge coupled to said sensor follower finger for generating a force signal indicative of the force exerted by said sensor follower finger in pressing against said tubing wall.

2. The apparatus of claim 1 wherein the processor is calibrated to the gauge during the part of the cycle when the sensor follower finger is not in contact with the tubing wall.

3. The apparatus of claim 2 wherein the processor is calibrated to the gauge during each cycle of the sensor follower finger during the part of the cycle when the sensor follower finger is not in contact with the tubing wall.

4. The apparatus of any one of the preceding claims wherein the gauge has a zero force value and the processor is calibrated to this zero force value during the part of the cycle when the sensor follower finger is not in contact with the tubing wall.

5. The apparatus of claim 4 wherein the gauge has a zero force value and the processor is calibrated to this zero force value during each cycle of the sensor follower finger during the part of the cycle when the sensor follower finger is not in contact with the tubing wall.

6. A method for measuring pressure within a compressible pumping segment (16) of a fluid line having a flexible tubing wall (12), said pumping segment being received in a peristaltic pump mechanism (10), said peristaltic pump mechanism having a rotating camshaft (24) and a plurality of cam follower fingers (23) journalled thereto and including an upstream end cam follower finger (30) and a downstream end cam follower finger (32) adapted to sequentially press against said pumping segment producing cyclically recurring upstream and downstream pressure communication modes of a duty cycle of said pump in which a central portion of said pumping segment is alternately in communication with only an upstream portion of said fluid line and only a downstream portion of said fluid line, respectively, characterized in the steps of:

periodically compressing said pumping segment with at least one finger member with a predetermined amount of displacement of said flexible tubing wall without occluding said fluid line;

measuring a force parameter exerted by said finger member on said flexible tubing wall and generating a force signal indicating said measured force parameter; and

determining fluid pressure within said tubing responsive to said force signal.

7. The method of Claim 6 wherein said pressure is determined from said force parameter, said amount of displacement, and a tube stiffness constant based upon the modulus of elasticity and thickness of the tubing wall.

8. The method of claims 6 or 7 wherein:

the step of measuring a force parameter comprises applying a gauge to the at least one finger member to measure said force parameter; and

the step of determining fluid pressure comprises the step of calibrating to the gauge during the part of the cycle when the at least one finger member is not compressing said tubing wall.

9. The method of any one of claims 6 to 8 wherein the step of calibrating during the part of the cycle when the at least one finger member is not compressing said tubing wall comprises calibrating to the gauge during each cycle.

10. The method of any one of claims 6 to 9 wherein:

the step of measuring a force parameter comprises applying a gauge to the at least one finger member to measure said force parameter, said gauge having a zero force value; and

the step of determining fluid pressure comprises the step of calibrating to this zero force value during the part of the cycle when the at least one finger member is not compressing the tubing wall.

11. The method of claim 10 wherein the step of calibrating to the zero force value includes calibrating to the zero force value during each cycle of the sensor follower finger during the part of the cycle when the sensor follower finger is not compressing the pumping segment.

**Patentansprüche**

1. Vorrichtung zum Messen des Druckes in einer Flüssigkeitsleitung mit einem Pumpensegment (16) mit einer elastischen Schlauchwand (12), umfassend einen peristaltischen Pumpenmechanismus (10) zur Aufnahme des Pumpensegments, wobei der peristaltische Pumpenmechanismus eine Mehrzahl peristaltischer Finger (23) aufweist, mit einem oberhalb liegenden Finger (30) und einem unterhalb liegenden Finger (32), die sequentiell gegen das Pumpensegment drücken, wodurch zyklisch wiederkehrende, obere und untere Druckverbindungsmodi eines Arbeitszykluses der Pumpe erzeugt werden, wobei ein Teilstück des Pumpsensegments zwischen den genannten Endfingern wechselnd in Verbindung mit nur einem oberen Teilstück der Flüssigkeitsleitung bzw. mit nur einem unteren Teilstück der Flüssigkeitsleitung steht,

einen Prozessor (53) zum Empfang eines Kraftsignals in dem oberen Druckverbindungsmodus und zum Empfang des Kraftsignals in dem unteren Druckverbindungsmodus, wobei der Prozessor basierend auf den Werten des Kraftsignals ein Verstopfungssignal erzeugt, dadurch gekennzeichnet, daß

sich zwischen dem oberen Endfinger und dem unteren Endfinger ein Sensorfolgefinger (36) befindet, um während eines Teils des Arbeitszykluses zyklisch gegen das Pumpensegment zu drücken, um die Schlauchwand des Segments um ein vorbestimmtes Ausmaß zu verschieben, ohne dabei die Flüssigkeitsleitung zu verstopfen, und umfassend

ein mit dem Sensorfolgefinger gekoppeltes Eichmaß, zur Erzeugung eines Kraftsignals, welches die Kraft anzeigt, die von dem Sensorfolgefinger durch Drücken gegen die Schlauchwand ausgeübt wird.

2. Vorrichtung nach Anspruch 1, dadurch gekennzeichnet, daß der Prozessor während des Teils des Zykluses, in dem sich der Sensorfolgefinger nicht in Kontakt mit der Schlauchwand befindet, auf das Eichmaß geeicht ist.

3. Vorrichtung nach Anspruch 2, dadurch gekennzeichnet, daß der Prozessor während jedem Zyklus des Sensorfolgefingers dann auf das Eichmaß geeicht ist, wenn sich der Sensorfolgefinger nicht in Kontakt mit der Schlauchwand befindet.

4. Vorrichtung nach einem der vorstehenden Ansprüche, dadurch gekennzeichnet, daß das Eichmaß einen Nullkraftwert aufweist und daß der Prozessor während dem Teil des Zykluses auf diesen Nullkraftwert geeicht ist, wenn sich der Sensorfolgefinger nicht in Kontakt mit der Schlauchwand befindet.

5. Vorrichtung nach Anspruch 4, dadurch gekennzeichnet, daß das Eichmaß einen Nullkraftwert aufweist und daß der Prozessor während jedem Zyklus des Sensorfolgefingers dann auf den Nullkraftwert geeicht ist, wenn sich der Sensorfolgefinger nicht in Kontakt mit der Schlauchwand befindet.

6. Verfahren zum Messen des Druckes in einem zusammendrückbaren Pumpensegment (16) einer Flüssigkeitsleitung mit einer elastischen Schlauchwand (12), wobei das Pumpensegment in einem peristaltischen Pumpenmechanismus (10) aufgenommen wird, wobei der peristaltische Pumpenmechanismus eine drehbare Nockenwelle (24) und eine Mehrzahl von Nockenfolgefingern (23) aufweist, welche daran lagern und welche einen oberen Nockenfolgefinger (30) und einen unteren Nockenfolgefinger (32) umfassen, welche sequentiell gegen das Pumpensegment drücken, wodurch sie zyklisch wiederkehrende, obere und untere Druckverbindungsmodi eines Arbeitszykluses der Pumpe erzeugen, wobei ein Mittelteil des Pumpensegments wechselweise in Verbindung mit nur einem oberhalb liegenden Teilstück der Flüssigkeitsleitung bzw. mit nur einem unterhalb liegenden Teilstück der Flüssigkeitsleitung steht, gekennzeichnet durch folgende Schritte:

periodisches Zusammendrücken des Pumpensegments mit wenigstens einem Fingerelement, mit einem vorbestimmten Ausmaß an Verschiebung der elastischen Schlauchwand, ohne die Flüssigkeitsleitung dabei zu verstopfen;

Messen eines durch das Fingerelement auf die elastische Schlauchwand ausgeübten Kraftparameters und Erzeugung eines Kraftsignals, das den gemessenen Kraftparameter anzeigt; und

Feststellen des Flüssigkeitsdrucks in dem Schlauch, als Reaktion auf das Kraftsignal.

7. Verfahren nach Anspruch 6, dadurch gekennzeichnet, daß der Druck durch den Kraftparameter, das Verschiebungsausmaß und eine Schlauchsteifheitskonstante, die auf dem Elastizitätsmodul und der Dicke der Schlauchwand basiert, ermittelt wird.

8. Verfahren nach Anspruch 6 oder 7, dadurch gekennzeichnet, daß:

der Schritt des Messens eines Kraftparameters die Anwendung eines Eichmaßes auf wenigstens ein Fingerelement umfaßt, um den Kaftparameter zu messen; und daß

der Schritt des Feststellens des Flüssigkeitsdrucks den Schritt der Eichung auf das Eichmaß umfaßt, und zwar während dem Teil des Zykluses, wenn das wenigstens eine Fingerelement die Schlauchwand nicht zusammendrückt.

9. Verfahren nach einem der Ansprüche 6 bis 8, dadurch gekennzeichnet, daß der Schritt der Eichung während dem Teil des Zykluses, wenn das wenigstens eine Fingerelement die Schlauchwand nicht zusammendrückt, die Eichung auf das Eichmaß während jedem Zyklus umfaßt.

10. Verfahren nach einem der Ansprüche 6 bis 9, dadurch gekennzeichnet, daß:

der Schritt des Messens eines Kraftparameters die Anwendung eines Eichmaßes auf das wenigstens eine Fingerelement umfaßt, um den Kraftparameter zu messen, wobei das Eichmaß einen Nullkraftwert aufweist; und daß

der Schritt des Feststellens des Flüssigkeitsdrucks den Schritt der Eichung auf diesen Nullkraftwert umfaßt, und zwar während dem Teil des Zykluses, wenn das wenigstens eine Fingerelement die Schlauchwand nicht zusammendrückt.

11. Verfahren nach Anspruch 10, dadurch gekennzeichnet, daß der Schritt der Eichung auf den Nullkraftwert die Eichung auf den Nullkraftwert während jedem Zyklus des Sensorfolgefingers während dem Teil des Zykluses umfaßt, wenn der Sensorfolgefinger das Pumpensegment nicht zusammendrückt.

**Revendications**

1. Appareil pour mesurer la pression à l'intérieur d'un conduit à liquide comportant un segment de pompage (16) muni d'une paroi de tue flexible (12), comprenant un mécanisme de pompe péristaltique (10) destiné à recevoir ledit segment de pompage, ledit mécanisme de pompe péristaltique ayant une multiplicité de doigts péristaltiques (23) comprenant un doigt terminal amont (30) et un doigt terminal aval (32) conçus pour exercer séquentiellement une pression contre ledit segment de pompage pour produire des modes de communication de pression amont et aval répétés cycliquement d'un cycle opératoire de ladite pompe dans lequel une partie dudit segment de pompage entre lesdits doigts terminaux est tour à tour en communication avec une partie amont seulement dudit conduit à liquide et avec une partie aval seulement dudit conduit à liquide respectivement, un processeur (53) destiné à recevoir un signal de force dans ledit mode de communication de pression amont et destiné à recevoir ledit signal de force dans ledit mode de communication de pression aval et conçu pour produire un signal d'occlusion sur la base des valeurs desdits signaux de force, un doigt suiveur de capteur (36) disposé entre ledit doigt terminal amont et ledit doigt terminal aval et conçu pour exercer cycliquement une pression contre ledit segment de pompage pendant une partie dudit cycle opératoire pour déplacer la paroi du tube dudit segment d'une quantité prédéterminée sans occlure le conduire à liquide, et une jauge couplée audit doigt suiveur de capteur pour produire un signal de force indiquant la force exercée par ledit doigt suiveur de capteur lorsqu'il exerce une pression contre ladite paroi du tube.

2. Appareil selon la revendication 1, dans lequel le processeur est étalonné à la jauge pendant la partie du cycle pendant laquelle le doigt suiveur de capteur n'est pas en contact avec la paroi du tube.

3. Appareil selon la revendication 2, dans lequel le processeur est étalonné à la jauge pendant chaque cycle du doigt suiveur de capteur pendant la partie du cycle pendant laquelle le doigt suiveur de capteur n'est pas en contact avec la paroi du tube.

4. Appareil selon l'une quelconque des revendications précédentes, dans lequel la jauge a une valeur de force zéro et le processeur est étalonné à cette valeur de force zéro pendant la partie du cycle pendant laquelle le doigt suiveur de capteur n'est pas en contact avec la paroi du tube.

5. Appareil selon la revendication 4, dans lequel la jauge a une valeur de force zéro et le processeur est étalonné à cette valeur de force zéro pendant chaque cycle du doigt suiveur de capteur pendant la partie du cycle pendant laquelle le doigt suiveur de capteur n'est pas en contact avec la paroi du tube.

6. Procédé pour mesurer la pression à l'intérieur d'un segment de pompage compressible (16) d'un conduit à liquide ayant une paroi de tube flexible (12), ledit segment de pompage étant reçu dans un mécanisme de pompe péristaltique (10), ledit mécanisme de pompe péristaltique ayant un arbre à cames rotatif (24) et une multiplicité de doigts suiveurs de cames (23) montés sur celui-ci et comprenant un doigt suiveur de carne terminal amont (30) et un doigt suiveur de came terminal aval (32) conçus pour exercer de manière séquentielle une pression contre ledit segment de pompage pour produire des modes de communication de pression amont et aval répétés cycliquement d'un cycle opératoire de ladite pompe dans lequel une partie centrale dudit segment de pompage est tour à tour en communication avec une partie amont seulement dudit conduit à liquide et avec une partie aval seulement dudit conduit à liquide, respectivement, caractérisé par les étapes consistant à :

comprimer périodiquement ledit segment de pompage avec au moins un élément formant doigt avec une quantité prédéterminée de déplacement de ladite paroi de tube flexible sans occlure ledit conduit à liquide;

mesurer un paramètre de force exercé par ledit élément formant doigt sur ladite paroi de tube flexible et produire un signal de force indiquant ledit paramètre de force mesuré ; et

déterminer la pression du liquide à l'intérieur dudit tube en réponse audit signal de force.

7. Procédé selon la revendication 6, dans lequel ladite pression est déterminée à partir dudit paramètre de force, de ladite quantité de déplacement et d'une constante de rigidité de tube basée sur le module d'élasticité et l'épaisseur de la paroi du tube.

8. Procédé selon la revendication 6 ou 7, dans lequel:

l'étape de mesure d'un paramètre de force comprend l'application d'une jauge à l'élément formant doigt ou aux éléments formant doigts pour mesurer ledit paramètre de force; et

l'étape de détermination de la pression du liquide comprend l'étape d'étalonnage à la jauge pendant la partie du cycle pendant laquelle le ou les éléments formant doigt ne compriment pas ladite paroi du tube.

9. Procédé selon l'une quelconque des revendications 6 à 8, dans lequel l'étape d'étalonnage pendant la partie du cycle pendant laquelle le ou les éléments formant doigt ne compriment pas ladite paroi du tube comprend l'étalonnage à la jauge pendant chaque cycle.

10. Procédé selon l'une quelconque des revendications 6 à 9, dans lequel :

l'étape de mesure d'un paramètre de force comprend l'application d'une jauge à l'élément formant doigt ou aux éléments formant doigts pour mesurer ledit paramètre de force, ladite jauge ayant une valeur de force zéro; et

l'étape de détermination de la pression du liquide comprend l'étape d'étalonnage à cette valeur de force zéro pendant la partie du cycle pendant laquelle le ou les éléments formant doigt ne compriment pas la paroi du tube.

11. Procédé selon la revendication 10, dans lequel l'étape d'étalonnage à la valeur de force zéro comprend l'étalonnage à la valeur de force zéro pendant chaque cycle du doigt suiveur de capteur pendant la partie du cycle pendant laquelle le doigt suiveur de capteur ne comprime pas le segment de pompage.

FIG. 1

# FIG. 2

# FIG. 4

FIG. 3

FIG. 5

RATIONAL PHASE INDICATOR — 70

FINGER #1 OCCLUDING? — 72

FINGER #2 OCCLUDING? — 74

SENSOR FOLLOWER CONTACT? — 76

CALIBRATE SENSOR — 78

MONITOR PRESSURE — 80

PRESSURE ABOVE MAXIMUM THRESHOLD? — 90

PRESSURE BELOW MINIMUM THRESHOLD? — 82

NO UPSTREAM OCCLUSION — 84

DOWN-STREAM OCCLUSION ERROR — 94

NO DOWNSTREAM ERROR — 92

UPSTREAM OCCLUSION ERROR — 86

ALARM — 88